# EUROPÄISCHE PATENTSCHRIFT

(11) **EP 3 272 732 B1**
(45) Veröffentlichungstag und Bekanntmachung des Hinweises auf die Patenterteilung: **18.09.2019**
(21) Anmeldenummer: 16180050.3
(22) Anmeldetag: 19.07.2016
(51) Int. Cl.: C07C 67/38, C07C 69/24

(54) **VERFAHREN ZUR ALKOXYCARBONYLIERUNG ETHYLENISCH UNGESÄTTIGTER VERBINDUNGEN MIT MONOPHOSPHINLIGANDEN**
METHOD FOR THE ALCOXY CARBONYLATION OF ETHYLENICALLY UNSATURATED COMPOUNDS WITH MONOPHOSPHIN LIGANDS
PROCÉDÉ D'ALKOXY-CARBONYLATION DE LIAISONS INSATURÉES EN ÉTHYLÈNE AVEC DES LIGANDS DE MONOPHOSPHINE

(43) Veröffentlichungstag der Anmeldung: 24.01.2018
(73) Patentinhaber: Evonik Degussa GmbH, 45128 Essen (DE)
(72) Erfinder: DONG, Kaiwu, 236800 Bo Zhou (CN); JACKSTELL, Ralf, 27478 Cuxhaven Altenwalde (DE); BELLER, Matthias, 18211 Ostseebad Nienhagen (DE); FRIDAG, Dirk, 45721 Haltern am See (DE); HESS, Dieter, 45770 Marl (DE); DYBALLA, Katrin Marie, 45657 Recklinghausen (DE); GEILEN, Frank, 45721 Haltern am See (DE); FRANKE, Robert, 45772 Marl (DE)

(56) Entgegenhaltungen:
- EP-A1- 0 282 142
- EP-A1- 0 441 446
- FANG X. ET AL.: "Palladium-Catalyzed Alkoxycarbonylation of Conjugated Dienes under Acid-Free Conditions: Atom-Economic Synthesis of beta,gamma-Unsaturated Esters", ANGEW. CHEM. INT. ED., Bd. 53, Nr. 34, 18. August 2014 (2014-08-18), Seiten 9030-9034, XP055330056,
- JIE LIU ET AL: "Ligand-Controlled Palladium-Catalyzed Alkoxycarbonylation of Allenes: Regioselective Synthesis of [alpha],[beta]- and [beta],[gamma]-Unsaturated Esters", JOURNAL OF THE AMERICAN CHEMICAL SOCIETY, Bd. 137, Nr. 26, 8. Juli 2015 (2015-07-08) , Seiten 8556-8563, XP055331891, US ISSN: 0002-7863, DOI: 10.1021/jacs.5b04052
- BRENNFÜHRER A. ET AL: "Palladium-Catalyzed Carbonylation Reaction of Alkenes and Alkynes", CHEMCATCHEM, Bd. 1, Nr. 1, 24. August 2009 (2009-08-24) , Seiten 28-41, XP055121332, DOI: 10.1002/cctc.200900062

## Beschreibung

Die vorliegende Erfindung betrifft ein neues Verfahren zur Alkoxycarbonylierung ethylenisch ungesättigter Verbindungen mit Monophosphinliganden.

Die Alkoxycarbonylierung ethylenisch ungesättigter Verbindungen ist ein Prozess mit steigender Bedeutung. Unter einer Alkoxycarbonylierung versteht man die Umsetzung von ethylenisch ungesättigten Verbindungen, wie Olefinen, mit Kohlenmonoxid und Alkoholen in Gegenwart eines Metalls bzw. eines Metallkomplexes und eines Liganden zu den entsprechenden Estern:

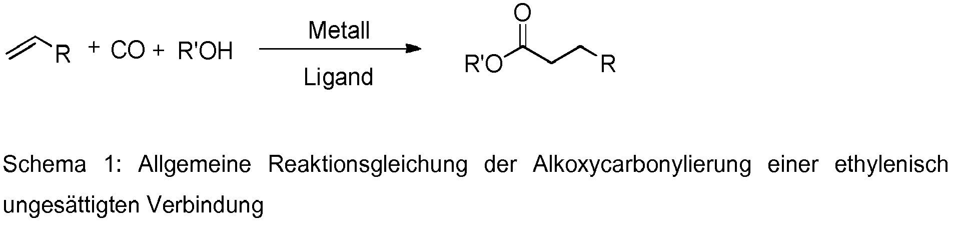

Unter den Alkoxycarbonylierungsreaktionen ist die Ethen-Methoxycarbonylierung zu 3-Methylpropionat von Bedeutung als Zwischenstufe für die Herstellung von Methylmethacrylat (S. G. Khokarale, E. J. García-Suárez, J. Xiong, U. V. Mentzel, R. Fehrmann, A. Riisager, Catalysis Communications 2014, 44, 73-75). Die Ethen-Methoxycarbonylierung wird in Methanol als Lösemittel bei milden Bedingungen mit einem durch Phosphinliganden modifizierten Palladiumkatalysator durchgeführt.

Die Alkoxycarbonylierung kann zu verzweigten (iso-) oder linearen (n-) Produkten führen. Somit ist neben der Ausbeute die n-/iso-Selektivität ein wichtiger Parameter bei der Entwicklung neuer katalytischer Systeme für die Alkoxycarbonylierung.

Es ist bekannt, Monophosphinverbindungen als Liganden für die Alkoxycarbonylierung einzusetzen. Ein Beispiel hierfür ist die Alkoxycarbonylierung von Isopren mit Benzylalkohol in Gegenwart eines Pd-Komplexes. Bei dieser Reaktion wurden beispielsweise unter Verwendung des unter dem Handelsnamen cataCXium PtB erhältlichen Liganden N-Phenyl-2-(di-tert-butylphosphino)pyrrol gute Ausbeuten erzielt (Fang X. et al., Angew. Chem. Int. Ed., 2014, 53, 9030-9034). Allerdings erzielt dieser Ligand eine nur geringe Selektivität. Ähnliche heteroarylsubstituierte Monophosphinverbindungen, nämlich N-Phenyl-2-(di-phenyl-phosphino)pyrrol und N-Phenyl-2-(di-cyclohexyl-phosphino)pyrrol, wurden ebenfalls untersucht, erzielen aber nur geringe Ausbeuten bei der Alkoxycarbonylierung von Isopren mit Benzylalkohol (Fang X. *et al*., aaO.). Die Umsetzung von Isopren mit aliphatischen Alkoholen wurde nicht untersucht.

Die vorliegende Erfindung hat die Aufgabe, ein neues Verfahren zur Alkoxycarbonylierung ethylenisch ungesättigter Verbindungen mit Monophosphinliganden bereitzustellen, mit dem sich eine hohe Ausbeute und hohe n/iso-Selektivität erzielen lassen. Insbesondere soll das Verfahren für die Alkoxycarbonylierung von langkettigen ethylenisch ungesättigten Verbindungen, beispielsweise C₈-Olefinen geeignet sein.

Diese Aufgabe wird gelöst durch ein Verfahren umfassend die Verfahrensschritte:
a) Vorlegen einer ethylenisch ungesättigten Verbindung;
b) Zugabe eines Monophosphinliganden und einer Verbindung, welche Pd umfasst, oder Zugabe eines Komplexes umfassend Pd und einen Monophosphinliganden;
c) Zugabe eines aliphatischen Alkohols;
d) Zuführen von CO;
e) Erwärmen des Reaktionsgemisches, wobei die ethylenisch ungesättigte Verbindung zu einem Ester umgesetzt wird;
wobei der Monophosphinligand ausgewählt ist aus Verbindungen gemäß einer der Formeln (**1**), (**2**), (**7**):

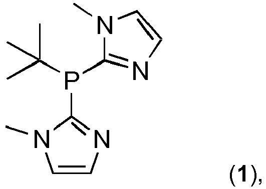

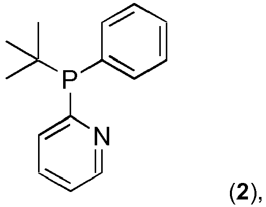

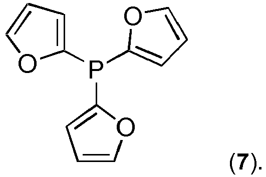

CO kann auch in mehreren Schritten zugeführt werden, so dass beispielsweise zunächst ein Teil des CO zugeführt, dann erwärmt und anschließend ein weiterer Teil CO zugeführt wird.

Die in dem erfindungsgemäßen Verfahren als Edukt eingesetzten ethylenisch ungesättigten Verbindungen enthalten eine oder mehrere Kohlenstoff-Kohlenstoff-Doppelbindungen. Diese Verbindungen werden auch im Folgenden zur Vereinfachung als Olefine bezeichnet. Die Doppelbindungen können terminal oder intern sein.

Bevorzugt sind ethylenisch ungesättigte Verbindungen mit 2 bis 30 Kohlenstoffatomen, bevorzugt 2 bis 22 Kohlenstoffatomen, besonders bevorzugt 2 bis 12 Kohlenstoffatomen.

In einer Ausführungsform umfasst die ethylenisch ungesättigte Verbindung 2 bis 30 Kohlenstoffatome, bevorzugt 6 bis 22 Kohlenstoffatome, besonders bevorzugt 8 bis 12 Kohlenstoffatome, am meisten bevorzugt 8 Kohlenstoffatome.

Die ethylenisch ungesättigten Verbindungen können zusätzlich zu der einen oder mehreren Doppelbindungen weitere funktionelle Gruppen enthalten. Vorzugsweise umfasst die ethylenisch ungesättigte Verbindung eine oder mehrere funktionelle Gruppen ausgewählt aus Carboxyl-, Thiocarboxyl-, Sulfo-, Sulfinyl-, Carbonsäureanhydrid-, Imid-, Carbonsäureester-, Sulfonsäureester-, Carbamoyl-, Sulfamoyl-, Cyano-, Carbonyl-, Carbonothioyl-, Hydroxyl-, Sulfhydryl- , Amino-, Ether-, Thioether-, Aryl-, Heteroaryl- oder Silylgruppen und/oder Halogensubstituenten.

In einer Ausführungsform umfasst die ethylenisch ungesättigte Verbindung keine weiteren funktionellen Gruppen außer Kohlenstoff-Kohlenstoff-Doppelbindungen.

In einer besonders bevorzugten Ausführungsform handelt es sich bei der ethylenisch ungesättigten Verbindung um ein unfunktionalisiertes Alken mit mindestens einer Doppelbindung und 2 bis 30 Kohlenstoffatomen, bevorzugt 6 bis 22 Kohlenstoffatomen, weiter bevorzugt 8 bis 12 Kohlenstoffatomen, am meisten bevorzugt 8 Kohlenstoffatomen.

Geeignete ethylenisch ungesättigte Verbindungen sind beispielsweise:
Ethen;
Propen;
C4-Olefine, wie 1-Buten, cis-2-Buten, trans-2-Buten, Gemisch von cis- und trans-2-Buten, Isobuten, 1,3-Butadien; Raffinat I bis III, Crack-C4
C5-Olefine, wie 1-Penten, 2-Penten, 2-Methyl-1-buten, 2-Methyl-2-buten, 2-Methyl-1,3-butadien (Isopren), 1,3-Pentadien;
C6-Olefine, wie Tetramethylethylen, 1,3-Hexadien, 1,3-Cyclohexadien;
C7-Olefine, wie 1-Methylcyclohexen, 2,4-Heptadien, Norbonadien;
C8-Olefine, wie 1-Octen, 2-Octen, Cycloocten, Di-n-buten, Di-iso-buten, 1,5-Cyclooctadien, 1,7-Octadien;
C9-Olefine, wie Tripropen;
C10-Olefine, wie Dicylcopentadien;
Undecene;
Dodecene;
interne C14-Olefine;
interne C15- bis C18-Olefine;
lineare oder verzweigte, cyclische, acyclische oder teilweise cyclische, interne C15- bis C30-Olefine;
Triisobuten, Tri-n-buten;
Terpene, wie Limonen, Geraniol, Farnesol, Pinen, Myrcen, Carvon, 3-Caren;
mehrfach ungesättigte Verbindungen mit 18 Kohlenstoffatomen, wie Linolsäure oder Linolensäure;
Ester ungesättigter Carbonsäuren, wie Vinylester von Essig- oder Propansäure, Alkylester ungesättigter Carbonsäuren, Methyl- oder Ethylester von Acrylsäure oder Methacrylsäure, Ölsäureester, Ölsäure Methyl- oder Ethylester, Ester der Linol- oder Linolensäure;
Vinylverbindungen, wie Vinylacetat, Vinylcyclohexen, Styrol, alpha-Methylstyrol, 2-Isopropenylnaphthalin;
2-Methyl-2-pentenal, Methyl-3-Pentenoat, Methacrylanhydrid.

In einer Variante des Verfahrens ist die ethylenisch ungesättigte Verbindung ausgewählt aus Propen, 1-Buten, cis- und/oder trans-2-Buten, oder Mischungen davon.

In einer Variante des Verfahrens ist die ethylenisch ungesättigte Verbindung ausgewählt aus 1-Penten, cis- und/oder trans-2-Penten, 2-Methyl-1-buten, 2-Methyl-2-buten, 3-Methyl-1-buten, oder Mischungen davon.

In einer bevorzugten Ausführungsform ist die ethylenisch ungesättigte Verbindung ausgewählt aus Ethen, Propen, 1-Buten, *cis-* und/oder *trans*-2-Buten, iso-Buten, 1,3-Butadien, 1-Penten, *cis-* und/oder *trans*-2-Penten, 2-Methyl-1-buten, 3-Methyl-1-buten, 2-Methyl-2-buten, Hexen, Tetramethylethylen, Hepten, n-Octen, 1-Octen, 2-Octen, oder Mischungen davon.

In einer Variante wird ein Gemisch ethylenisch ungesättigter Verbindungen eingesetzt. Als Gemisch wird im Sinne dieser Erfindung eine Zusammensetzung bezeichnet, die wenigsten zwei verschiedene ethylenisch ungesättigte Verbindungen enthält, wobei der Anteil jeder einzelnen ethylenisch ungesättigten Verbindungen vorzugsweise wenigstens 5 Gewichts-% bezogen auf das Gesamtgewicht des Gemisches beträgt.

Vorzugsweise wird ein Gemisch ethylenisch ungesättigter Verbindungen mit jeweils 2 bis 30 Kohlenstoffatomen, bevorzugt 4 bis 22 Kohlenstoffatomen, besonders bevorzugt 6 bis 12 Kohlenstoffatomen, am meisten bevorzugt 8 bis 10 Kohlenstoffatomen eingesetzt.

Geeignete Gemische ethylenisch ungesättigter Verbindungen sind die sogenannten Raffinate I bis III. Raffinat I umfasst 40 bis 50 % iso-Buten, 20 bis 30 % 1-Buten, 10 bis 20 % cis- und trans-2-Buten, bis zu 1 % 1,3-Butadien und 10 bis 20 % n-Butan und Isobutan. Das Raffinat II ist ein Teil der beim Naphthacracken entstehenden C₄-Fraktion und besteht im Wesentlichen aus den isomeren *n*-Butenen, Isobutan und *n*-Butan nach Abtrennung von Isobuten aus Raffinat I. Raffinat III ist ein Teil der beim Naphthacracken entstehenden C₄-Fraktion und besteht im Wesentlichen aus den isomeren *n*-Butenen und *n*-Butan.

Ein weiteres geeignetes Gemisch ist Di-n-Buten, auch bezeichnet als Dibuten, DNB oder DnB. Di-n-Buten ist ein Isomerengemisch von C8-Olefinen, das aus der Dimerisierung von Gemischen aus 1-Buten, cis-2-Buten und trans-2-Buten entsteht. Technisch werden der Regel Raffinat II oder Raffinat III-Ströme einer katalytischen Oligomerisierung unterworfen, wobei die enthaltenen Butane (n/iso) unverändert hervorgehen und die enthaltenen Olefine ganz oder teilweise umgesetzt werden. Neben dem dimeren Di-n-Buten, entstehen in der Regel auch höhere Oligomere (Tributen C12, Tetrabuten C16), die nach der Reaktion destillativ abgetrennt werden. Diese können ebenfalls als Edukte eingesetzt werden.

In einer bevorzugten Variante wird ein Gemisch umfassend iso-Buten, 1-Buten, cis- und trans-2-Buten eingesetzt. Vorzugsweise umfasst das Gemisch 1-Buten, cis- und trans-2-Buten.

Die erfindungsgemäße Alkoxycarbonylierung wird durch einen Pd-Komplex katalysiert. Der Pd-Komplex kann dabei in Verfahrensschritt b) entweder als präformierter Komplex umfassend Pd und den Monophosphinliganden zugegeben werden, oder *in situ* aus einer Verbindung, welche Pd umfasst, und dem freien Monophosphinliganden gebildet werden. Dabei wird die Verbindung, welche Pd umfasst, auch als Katalysatorvorstufe bezeichnet.

Die präformierten Komplexe können außerdem weitere Liganden umfassen, die an das Metallatom koordinieren. Dabei handelt es sich beispielsweise um ethylenisch ungesättigte Verbindungen oder Anionen. Geeignete zusätzliche Liganden sind beispielsweise Styrol, Acetat-Anionen, Maleinimide (z.B. N-Methylmaleinimid), 1,4-Naphthochinon, Trifluoroacetat-Anionen oder Chloridanionen.

In dem Fall, dass der Katalysator *in situ* gebildet wird, kann der Ligand im Überschuss zugegeben werden, so dass im Reaktionsgemisch auch ungebundener Ligand vorliegt.

Auch im Falle des Komplexes, der gleich zu Beginn zugesetzt wird, kann zusätzlich weiterer Ligand zugegeben werden, so dass im Reaktionsgemisch auch ungebundener Ligand vorliegt.

In einer Variante ist die Verbindung, welche Pd umfasst, ausgewählt aus Palladiumdichlorid (PdCl₂), Palladium(II)-acetylacetonat [Pd(acac)₂], Palladium(II)-acetat [Pd(OAc)₂], Dichloro(1,5-cyclooctadiene)palladium(II) [Pd(cod)₂Cl₂], Bis(dibenzylideneaceton)palladium [Pd(dba)₂], Bis(acetonitrile)dichloropalladium(II) [Pd(CH₃CN)₂Cl₂], Palladium(cinnamyl)dichlorid [Pd(cinnamyl)Cl₂],

Vorzugsweise handelt es sich bei der Verbindung, welche Pd umfasst, um PdCl₂, Pd(acac)₂ oder Pd(OAc)₂. Besonders geeignet ist PdCl₂.

Der aliphatische Alkohol in Verfahrensschritt c) kann verzweigt oder linear sein, cyclisch, alicyclisch oder teilweise zyklisch und stellt insbesondere ein C₁- bis C₃₀-Alkanol dar. Es können Monoalkohole oder Polyalkohole verwendet werden.

Als aliphatischer Alkohol wird im Rahmen dieser Erfindung ein Alkohol bezeichnet, der keine aromatischen Gruppen umfasst, also beispielsweise ein Alkanol, Alkenol oder Alkinol.

Der Alkohol in Verfahrensschritt c) umfasst vorzugsweise 1 bis 30 Kohlenstoffatome, bevorzugt 1 bis 22 Kohlenstoffatome, besonders bevorzugt 1 bis 12 Kohlenstoffatome. Es kann sich dabei um einen Monoalkohol oder einen Polyalkohol handeln.

Der Alkohol kann zusätzlich zu der einen oder mehreren Hydroxylgruppen weitere funktionelle Gruppen enthalten. Vorzugsweise kann der Alkohol zusätzlich eine oder mehrere funktionelle Gruppen ausgewählt aus Carboxyl-, Thiocarboxyl-, Sulfo-, Sulfinyl-, Carbonsäureanhydrid-, Imid-, Carbonsäureester-, Sulfonsäureester-, Carbamoyl-, Sulfamoyl-, Cyano-, Carbonyl-, Carbonothioyl-, Sulfhydryl- , Amino-, Ether-, Thioether-, oder Silylgruppen, und/oder Halogensubstituenten enthalten.

In einer Ausführungsform umfasst der Alkohol keine weiteren funktionellen Gruppen außer Hydroxylgruppen.

In einer Variante des Verfahrens ist der Alkohol im Verfahrensschritt c) ausgewählt aus der Gruppe der Monoalkohole.

In einer Variante des Verfahrens ist der Alkohol im Verfahrensschritt c) ausgewählt aus: Methanol, Ethanol, 1-Propanol, Iso-Propanol, Iso-Butanol, tert-Butanol, 1-Butanol, 2-Butanol, 1-Pentanol, 2-Pentanol, 3-Pentanol, 1-Hexanol, Cyclohexanol, 2-Ethylhexanol, Isononanol, 2-Propylheptanol.

In einer bevorzugten Variante ist der Alkohol in Verfahrensschritt c) ausgewählt ist aus Methanol, Ethanol, 1-Propanol, 1-Butanol, 1-Pentanol, 1-Hexanol, 2-Propanol, *tert*-Butanol, 3-Pentanol, Cyclohexanol, oder Mischungen davon.

In einer Variante des Verfahrens ist der Alkohol im Verfahrensschritt c) ausgewählt aus der Gruppe der Polyalkohole.

In einer Variante des Verfahrens ist der Alkohol im Verfahrensschritt c) ausgewählt aus: Diolen, Triolen, Tetraolen.

In einer Variante des Verfahrens ist der Alkohol im Verfahrensschritt c) ausgewählt aus: Cyclohexan-1,2-diol, 1,2-Ethandiol, 1,3-Propandiol, Glycerol, 1,2,4-Butantriol,2-Hydroxymethyl-1,3-Propandiol, 1,2,6-Trihydroxyhexan, Pentaerythritol, 1,1,1-Tri(hydroxymethyl)ethan.

In einer Variante des Verfahrens ist der Alkohol im Verfahrensschritt c) ausgewählt aus: Sucrose, Fructose, Mannose, Sorbose, Galactose und Glucose.

In einer bevorzugten Variante des Verfahrens ist der Alkohol im Verfahrensschritt c) ausgewählt aus Methanol, Ethanol, 1-Propanol, 1-Butanol, 1-Pentanol, 1-Hexanol.

In einer besonders bevorzugten Variante des Verfahrens ist der Alkohol im Verfahrensschritt c) ausgewählt aus: Methanol, Ethanol.

In einer besonders bevorzugten Variante des Verfahrens ist der Alkohol im Verfahrensschritt c) Methanol.

In einer Variante des Verfahrens wird der Alkohol im Verfahrensschritt c) im Überschuss eingesetzt.

In einer Variante des Verfahrens wird der Alkohol im Verfahrensschritt c) gleichzeitig als Lösungsmittel eingesetzt.

In einer Variante des Verfahrens wird ein weiteres Lösungsmittel verwendet, ausgewählt aus: Toluol, Xylol, Tetrahydrofuran (THF) oder Methylenchlorid (CH₂Cl₂).

CO wird in Schritt d) vorzugsweise bei einem CO-Partialdruck zwischen 0,1 und 10 MPa (1 bis 100 bar), bevorzugt zwischen 1 und 8 MPa (10 bis 80 bar), besonders bevorzugt zwischen 2 und 4 MPa (20 bis 40 bar) zugeführt.

Die Reaktionsmischung wird Schritt e) des erfindungsgemäßen Verfahrens vorzugsweise auf eine Temperatur zwischen 10 °C und 180 °C, bevorzugt zwischen 20 und 160 °C, besonders bevorzugt zwischen 40 und 120 °C erwärmt, um die ethylenisch ungesättigte Verbindung zu einem Ester umzusetzen.

Das molare Verhältnis von der in Schritt a) vorgelegten ethylenisch ungesättigten Verbindung zu dem in Schritt c) zugegebenen Alkohol beträgt vorzugsweise zwischen 1:1 bis 1:20, bevorzugt 1:2 bis 1:10, besonders bevorzugt 1:3 bis 1:4.

Das Massenverhältnis von Pd zu der in Schritt a) vorgelegten ethylenisch ungesättigten Verbindung beträgt vorzugsweise zwischen 0,001 und 0,5 Gew.-%, bevorzugt zwischen 0,01 und 0,1 Gew.-%, besonders bevorzugt zwischen 0,01 und 0,05 Gew.-%.

Das molare Verhältnis des Monophosphinliganden zu Pd beträgt vorzugsweise zwischen 0,1:1 und 400:1, bevorzugt zwischen 0,5:1 und 400:1, besonders bevorzugt zwischen 1:1 und 100:1, am meisten bevorzugt zwischen 2:1 und 50:1.

Vorzugsweise wird das Verfahren unter Zusatz einer Säure durchgeführt. In einer Variante umfasst das Verfahren deshalb zusätzlich den Schritt c'): Zugabe einer Säure zum Reaktionsgemisch. Dabei kann es sich vorzugsweise um eine Brønsted- oder eine Lewis-Säure handeln.

Geeignete Brønsted-Säuren haben vorzugsweise eine Säurestärke von pKₛ ≤ 5, bevorzugt eine Säurestärke von pKₛ ≤ 3. Die angegebene Säurestärke pKₛ bezieht sich auf den bei Normalbedingungen (25°C, 1,01325 bar) bestimmten pKₛ-Wert. Bei einer mehrprotonigen Säure bezieht sich die Säurestärke pKₛ im Rahmen dieser Erfindung auf den pKₛ-Wert des ersten Protolyseschrittes.

Vorzugsweise ist die Säure keine Carbonsäure.

Geeignete Brønsted-Säuren sind beispielsweise Perchlorsäure, Schwefelsäure, Phosphorsäure, Methylphosphonsäure und Sulfonsäuren. Vorzugsweise handelt es sich bei der Säure um Schwefelsäure oder eine Sulfonsäure. Geeignete Sulfonsäuren sind beispielsweise Methansulfonsäure, Trifluormethansulfonsäure, tert-Butansulfonsäure, p-Toluolsulfonsäure (PTSA), 2-Hydroxypropan-2-sulfonsäure, 2,4,6-Trimethylbenzolsulfonsäure und Dodecylsulfonsäure. Besonders bevorzugte Säuren sind Schwefelsäure, Methansulfonsäure, Trifluormethansulfonsäure und p-Toluolsulfonsäure.

Als Lewis-Säure kann beispielsweise Aluminiumtriflat eingesetzt werden.

In einer Ausführungsform beträgt die Menge an in Schritt c') zugesetzter Säure 0,3 bis 40 mol-%, bevorzugt 0,4 bis 15 mol-%, besonders bevorzugt 0,5 bis 5 mol-%, am meisten bevorzugt 0,6 bis 3 mol-%, bezogen auf die Stoffmenge der in Schritt a) eingesetzten ethylenisch ungesättigten Verbindung.

### Beispiele

Die folgenden Beispiele veranschaulichen die Erfindung.

### Allgemeine Arbeitsvorschriften

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet (Purification of Laboratory Chemicals, W. L. F. Armarego (Autor), Christina Chai (Autor), Butterworth Heinemann (Elsevier), 6. Auflage, Oxford 2009).

Phosphortrichlorid (Aldrich) wurde vor dem Einsatz unter Argon destilliert. Alle präparativen Arbeiten erfolgten in ausgeheizten Gefäßen. Die Charakterisierung der Produkte erfolgte mittels NMR-Spektroskopie. Chemische Verschiebungen (δ) werden in ppm angegeben. Die Referenzierung der ³¹P-NMR-Signale erfolgte gemäß: SR_{31P} = SR_{1H} ^{∗} (BF_{31P} / BF_{1H}) = SR_{1H} ^{∗} 0,4048. (Robin K. Harris, Edwin D. Becker, Sonia M. Cabral de Menezes, Robin Goodfellow, und Pierre Granger, Pure Appl. Chem., 2001, 73, 1795-1818; Robin K. Harris, Edwin D. Becker, Sonia M. Cabral de Menezes, Pierre Granger, Roy E. Hoffman und Kurt W. Zilm, Pure Appl. Chem., 2008, 80, 59-84).

Die Aufnahme von Kernresonanzspektren erfolgte an Bruker Avance 300 bzw. Bruker Avance 400, die gaschromatografische Analyse an Agilent GC 7890A, die Elementaranalyse an Leco TruSpec CHNS und Varian ICP-OES 715, und die ESI-TOF Massenspektrometrie an Thermo Electron Finnigan MAT 95-XP und Agilent 6890 N/5973 Geräten.

### Darstellung von Chlor-2-pyridyl-tert-butylphosphin (Vorstufe A)

Der Grignard für die Synthese von Chlor-2-pyridyl-t-butylphosphin wird nach der "Knochelmethode" mit Isopropylmagnesiumchlorid dargestellt (Angew. Chem. 2004, 43, 2222-2226). Die Aufarbeitung erfolgt nach der Methode von Budzelaar (Organometallics 1990, 9, 1222-1227).

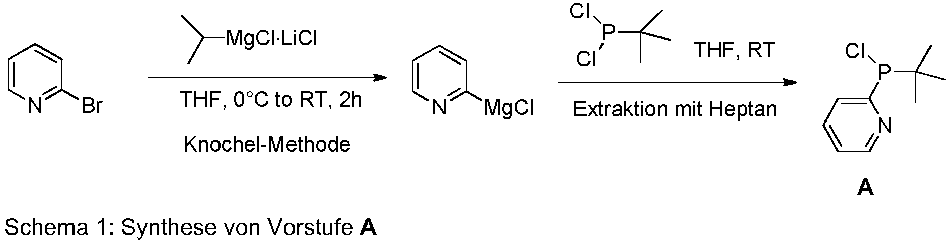

In einen 50 ml-Rundkolben mit Magnetrührer und Septum werden unter Argon 8,07 ml einer 1,3 M Isopropylmagnesium-chloridlösung (Knochel-Reagenz) gegeben und auf -15 °C gekühlt. Danach werden zügig 953.5 µl (10 mmol) 2-Bromopyridin zugetropft. Die Lösung wird sofort gelb. Man lässt auf-10 °C aufwärmen. Der Umsatz der Reaktion wird wie folgt bestimmt: man entnimmt ca. 100 µl Lösung und gibt sie in 1 ml einer gesättigten Ammoniumchloridlösung. Wenn die Lösung "sprudelt", dann hat sich noch nicht viel Grignard gebildet. Die wässrige Lösung wird mit einer Pipette Ether extrahiert und die organische Phase über Na₂SO₄ getrocknet. Von der etherischen Lösung wird ein GC aufgenommen. Wenn sich viel Pyridin gebildet hat im Vergleich zu 2-Bromopyridin, dann hat man hohe Umsätze. Bei -10 °C hat sich wenig umgesetzt. Nach Aufwärmen auf Raumtemperatur und 1-2 stündigen Rühren wird die Reaktionslösung braungelb. Ein GC-Test zeigt vollständigen Umsatz. Jetzt kann die Grignardlösung zu einer Lösung von 1,748 g (11 mmol) Dichloro-tert-butylphosphin in 10 ml THF, die vorher auf -15 °C gekühlt worden ist, mit einer Spritzenpumpe langsam zugetropft werden. Wichtig ist, dass die Dichloro-tert-butylphosphinlösung gekühlt wird. Bei Raumtemperatur würde man erhebliche Mengen an Dipyridyl-tert-butylphosphin erhalten. Es entsteht anfangs eine klare gelbe Lösung, die dann trübe wird. Man lässt auf Raumtemperatur aufwärmen und über Nacht Rühren. Laut GCMS hat sich viel Produkt gebildet. Man entfernt das Lösungsmittel im Hochvakuum und erhält einen weißlichen Feststoff, der braune Stellen enthält. Der Feststoff wird mit 20 ml Heptan suspendiert und der Feststoff wird im Ultraschallbad zerkleinert. Nach dem Absetzen lassen des weißen Feststoffes wird die Lösung dekantiert. Der Vorgang wird zwei Mal mit je 10-20 ml Heptan wiederholt. Nach dem Einengen der Heptanlösung im Hochvakuum wird diese unter Vakuum destilliert. Bei 4,6 mbar, 120 °C Ölbad und 98 °C Übergangstemperatur kann das Produkt destilliert werden. Man erhält 1,08 g eines farblosen Öls. (50%).

Analytische Daten: ¹H NMR (300 MHz, C₆D₆): δ 8,36 (m, 1H, Py), 7,67 (m, 1H, Py), 7,03-6,93 (m, 1H, Py), 6,55-6,46 (m, 1H, Py), 1,07 (d, J = 13,3 Hz, 9H, t-Bu).
¹³C NMR (75 MHz, C₆D₆): δ 162,9, 162,6, 148,8, 135,5, 125,8, 125,7, 122,8, 35,3, 34,8, 25,9 und 25,8.
³¹P NMR (121 MHz, C₆D₆) δ 97,9.
MS (EI) *m*:*z* (relative Intensität) 201 (M⁺,2), 147(32), 145 (100), 109 (17), 78 (8), 57,1 (17).

### Herstellung von Verbindung 1

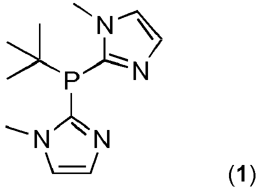

0,78 g (9,5 mmol) 1-Methylimidazol werden in einem 50 ml-Dreihalskolben mit Thermometer und Tropftrichter unter Argon eingewogen und in 10 ml THF gelöst. Dann gibt man 1,6 ml TMEDA zu der Lösung. Das Gemisch wird dann auf -78 °C heruntergekühlt. Danach werden 6 ml 1,6 N n-Butyllithium in Hexan mittels Tropftrichter zugetropft. Den 50 ml-Kolben mit der Reaktionsmischung lässt man 30 min bei Raumtemperatur rühren. Anschließend löst man 1,5 g tert-Butyldichlorphosphin in 20 ml THF. Man tropft dann das 1-Methylimidazol-BuLi-Gemisch bei -78 °C zum tert-Butyldichlorphosphin. Danach wird auf Raumtemperatur erwärmt. Es fällt ein Produkt aus. Die Suspension wird filtriert und der Rückstand wird in Wasser gelöst und anschließend dreimal mit Dichlormethan gewaschen. Die organische Phase wird mit Na₂SO₄ getrocknet und dann das Lösungsmittel im Vakuum entfernt. Den Rückstand löst man mit 5 ml Dichlormethan und überschichtet mit 20 ml Diethylether. Es kristallisiert das Produkt. Das Produkt konnte in 0,8 g erhalten werden.
Reinheit (NMR) = 98%,
³¹P NMR (CD₂Cl₂, 121 MHz) = -32,25 ppm,
¹³C NMR(CD₂Cl₂, 75 MHz) = 144 s, 130,2 d (J_{PC} = 3,7 Hz), 123,8 s, 34,2 d, (J_{PC} = 11,7 Hz), 25,9 d, (J_{PC} = 14,3 Hz)
¹H NMR (CD₂Cl₂, 300 MHz,): 7,04, d, (J = 1 Hz, 1H), 6,94 dd (J = 1 Hz, J = 1,5 Hz, 1H), 3,4 s (6H), 1,2 d (J = 14,6 Hz, 9H)
HRMS: berechnet für C₁₂H₁₉N₄P: 251,14201, gefunden: 251,14206

### Herstellung von 2-(tert-Butyl(phenyl)phosphino)pyridin (Verbindung 2)

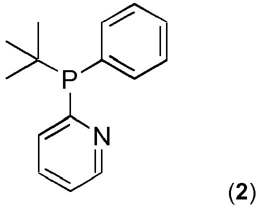

3,4 g (16,8 mmol) 2-(tert-Butylchlorphosphino)pyridin werden unter Argon in einem 100 ml-Dreihalskolben versehen mit Tieftemperaturthermometer und Magnetrührer in 50 ml absolutem Diethylether gelöst. Es wird auf -78 °C heruntergekühlt. Bei dieser Temperatur werden innerhalb von 10 Minuten 10 ml einer 1,8 N Phenyllithiumlösung (in Dibutylether) mittels eines Tropftrichters zugesetzt. Es wird 10 Minuten bei dieser Temperatur gerührt und anschließend auf Raumtemperatur erwärmt und noch eine weiter halbe Stunde gerührt. Diese Lösung wird dreimal mit 10 ml entgastem Wasser gewaschen. Die organische Phase wird nun im Feinvakuum von 10⁻¹ Torr destilliert. Das Produkt wird bei diesem Druck bei 130 °C als klare Flüssigkeit in hoher Reinheit von größer 97 % (NMR) erhalten. Die Ausbeute beträgt 3,85 g (93%).

### Analytik:

³¹P (Aceton-d₆, 121 MHz), 16,31 s,
¹³C (75 MHz, Acetond₆, 165,1 (d, J_{PC} = 10,5 Hz), 150,3 (d, J_{PC} = 5 Hz), 137,3 s, 137,0 s, 136,7 s, 135,9 d, 135,9 (d, J_{PC} = 7,6 Hz), 131,1 s, 130,6 s, 130,2 s, 128,9 (d, J_{PC} = 8 Hz), 122,9 s, 32,1 (d, J_{PC} = 13,1 Hz), 28,5 (d, J_{PC} = 13,7 Hz),
¹H (Aceton-d₆, 300 MHz):
8,74 (dm, J = 4,7 Hz), 7,7-7,6 m (2 H), 7,4-7,3 (m, 3 H), 7,28-7,23 (m, 1 H), 1,2 (d, J = 12,6 Hz, 9 H)
MS (EI, 70 eV): *m*/*z* (%), 243 (M+, 17), 203 (65), 187 (78), 156 (6), 126(8), 109(100), 78(11), 57(11), HRMS(EI), berechnet für C15H18N1P1: 243.11714, gefunden: 243,11753

### Weitere Liganden

Die folgenden Verbindungen sind kommerziell verfügbar bzw. auf bekanntem Wege herstellbar.

| | |
|---|---|
| 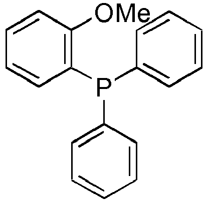 | **3** (VB) |
| 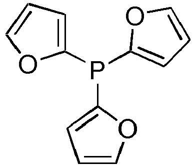 | **7** |
| 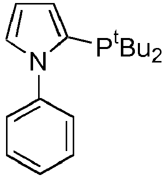 | **10** (VB) |

| | |
|---|---|
| VB: Vergleichsbeispiel | |

### Alkoxycarbonylierungsversuche

### Allgemeine Versuchsbeschreibung für Reaktionen im Batchversuch

Die entsprechenden Mengen an Substrat, Palladiumsalz, Säure und Alkohol werden unter Argon und Magnetrührung in einem 50 ml-Schlenkgefäß vermischt.

Ein 100 ml Stahlautoklav der Firma Parr versehen mit einem Gaseinlass und einem Gasauslassventil, einem digitalem Druckaufnehmer, einem Temperaturfühler und einem Kugelhahn sowie einer eingebauten Kapillare zur Probennahme wird dreimal mittels Vakuum und Argonspülung von Sauerstoff befreit. Anschließend wird die Reaktionslösung aus dem Schlenkgefäß mittels einer Kapillare in den Autoklaven im Argongegenstrom durch den Kugelhahn befüllt. Anschließend wird entweder bei Raumtemperatur die entsprechende Menge an CO aufgepresst und dann auf Reaktionstemperatur hochgeheizt (Reaktionen, die nicht unter konstantem Druck gefahren werden) oder es wird erst auf Reaktionstemperatur hochgeheizt und dann wird das CO über eine Bürette, die mittels eines Druckminderers mit dem Autoklaven verbunden ist, aufgepresst. Diese Bürette wird dann noch auf ca. 100 bar mit CO befüllt und liefert während der Reaktion das benötigte CO bei einem konstanten Druck nach. Diese Bürette hat ein Totvolumen von ca. 30 ml und ist mit einem digitalen Druckaufnehmer versehen. Dann wird die Reaktion bei der benötigten Temperatur die entsprechende Zeit unter Rührung durchgeführt. Hierbei werden mittels einer Software (Specview der Firma SpecView Corporation) und einem Prozesscontroller der Firma Parr 4870 sowie einem 4875 Powercontroler Daten über den Druckverlauf im Autoklaven und in der Gasbürette aufgezeichnet. Falls benötigt, werden über die Kapillare über die GC Proben gesammelt und analysiert. Hierzu wird vor der Reaktion eine geeignete exakte Menge (2-10 ml) Isooctan als interner Standard mit in das Schlenkgefäß gegeben. Diese geben auch Auskunft über den Reaktionsverlauf. Am Ende der Reaktion wird der Autoklav auf Raumtemperatur heruntergekühlt, der Druck vorsichtig abgelassen, falls nötig Isooctan als interner Standard hinzugefügt und eine GC-Analyse bzw. bei neuen Produkten auch eine GC MS Analyse durchgeführt.

### Allgemeine Versuchsvorschrift für Autoklavenversuche in Glasvials

Es wird ein 300ml Parrreaktor verwendet. Diesem angepasst ist ein selbstgefertigter Aluminiumblock entsprechender Dimension welcher zu Heizen mittels handelsüblicher Magnetrührer z.B. der Firma Heidolph geeignet ist. Für das Innere des Autoklaven wurde eine runde Metallplatte der Stärke von ca. 1,5 cm angefertigt die 6 Bohrungen enthält, die dem Außendurchmesser der Glasvials entsprechen. Diesen Glasvials angepasst werden sie mit kleinen Magnetrührern bestückt. Diese Glasvials werden mit Schraubkappen und geeigneten Septen versehen und mit einer in der Glasbläserei angefertigten Spezialapparatur unter Argon mit den entsprechenden Reaktanden, Lösungsmittel und Katalysatoren und Additiven befüllt. Hierzu werden 6 Gefäße gleichzeitig befüllt, dies ermöglicht die Durchführung von 6 Reaktionen bei gleicher Temperatur und gleichem Druck in einem Experiment. Dann werden diese Glasgefäße mit Schraubkappen und Septen geschlossen und es wird jeweils ein kleine Spritzenkanüle geeigneter Größe durch die Septen gestochen. Dies ermöglicht später in der Reaktion den Gasaustausch. Diese Vials werden nun in der Metallplatte platziert und diese wird unter Argon in den Autoklaven überführt. Der Autoklav wird mit CO gespült und bei Raumtemperatur mit dem vorgesehenen CO-Druck befüllt. Dann wird mittels dem Magnetrührer unter Magnetrührung auf Reaktionstemperatur erhitzt und die Reaktion die entsprechende Zeit durchgeführt. Anschließend wird auf Raumtemperatur heruntergekühlt und der Druck langsam abgelassen. Anschließend wird der Autoklav mit Stickstoff gespült. Die Vials werden dem Autoklaven entnommen und mit einer definierten Menge eines geeigneten Standards versetzt. Es erfolgt eine GC Analyse mit deren Ergebnissen Ausbeuten und Selektivitäten bestimmt wurden.

### Analytik:

### Analytik Methanol

Methanol wurde in einer Lösungsmitteltrocknungsanlage vorbehandelt: Pure Solv MD-/ Lösungsmittel Purification System, Firma Innovative Technology Inc. One Industrial Way, Amesbury MA 01013

### Wasserwerte:

Bestimmt mit Karl Fischer Tritration: TitraLab 580-TIM580, Firma Radiometer Analytical SAS (Karl- Fischer Titration), Wassergehalt: Messbereiche, 0,1-100% w/w, gemessener Wassergehalt: 0,13889%

### Eingesetzt wurden:

Technisches Methanol Applichem: Nr. A2954,5000, Chargennummer: LOT: 3L005446 Wassergehalt max. 1 %
Methanol Acros Organics (über Molsieb): Wassergehalt 0,005 %, Codenummer: 364390010, Chargennummer: LOT 1370321

### Methoxycarbonylierung von Ethen

Ein 50 ml Schlenkgefäß wurde mit Pd(acac)₂ (6,53 mg, 0,04 mol%), Ligand (0,16 mol%), Ethen (1,5 g, 53 mmol), 20ml Methanol und para-Toluolsulfonsäure (PTSA, 61 mg, 0,6 mol%) beladen. Das Reaktionsgemisch wurde mittels einer Kapillare im Argongegenstrom in einen 100 ml-Stahl-Autoklaven wie oben beschrieben überführt. Der CO-Druck wurde auf 40 bar eingestellt. Die Reaktion lief 3 Stunden bei 80°C. Nach Abschluss der Reaktion wurde der Autoklav auf Raumtemperatur abgekühlt und vorsichtig entspannt. Isooctan (100 µl) wurde als interner GC-Standard zugesetzt. Ausbeute und Regioselektivität wurden mittels GC bestimmt.

Die Ergebnisse sind in der nachfolgenden Tabelle dargestellt.

| Ligand | Ausbeute |
|---|---|
| **1** | 30% |
| **2** | 14% |
| **3** (VB) | 3% |
| **7** | 18% |
| **10** (VB) | 0% |

| | |
|---|---|
| VB: Vergleichsbeispiel | |

Die erfindungsgemäßen Liganden **1, 2** und **7** erzielen deutlich bessere Ausbeuten bei der Methoxycarbonylierung von Ethen als die Vergleichsliganden **3** und **10.**

### Isomerisierende regioselektive Methoxycarbonylierung von 1-Octen

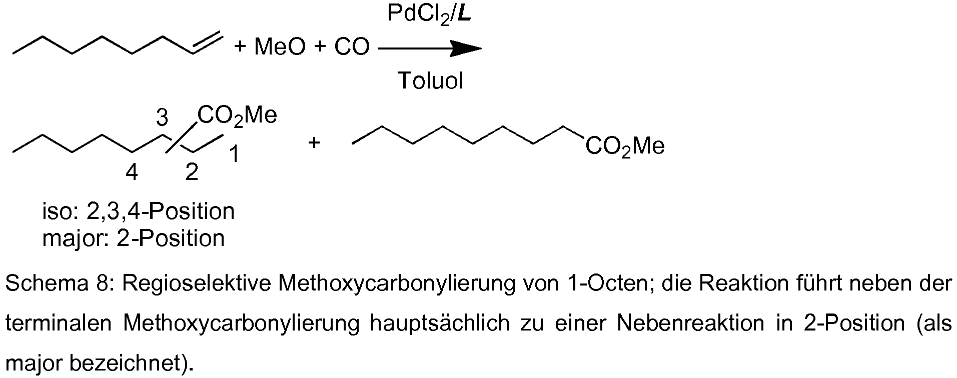

Das im Folgenden angegebene iso/n-Verhältnis gibt das Verhältnis von innenständig zu Estern umgesetzten Olefinen zu endständig zu Estern umgesetzten Olefinen an.

Ein 25 mL Schlenkgefäß wurde mit [Pd(acac)₂] (1.95 mg, 0.04 mol%), p-Toluolsulfonsäure (PTSA) (18.24 uL, 0.6 mol%) und MeOH (10 mL) befüllt. Ein 4 mL-Fläschchen wurde mit dem Liganden (0.16 mol%) befüllt und ein Magnetrührstab hinzugegeben. Danach wurden 1.25 mL der klaren gelben Lösung aus dem Schlenkgefäß und 1-Octen (315 µL, 2mmol) mit einer Spritze injiziert. Das Fläschchen wurde auf einen Probenhalter gesetzt, der wiederum unter Argon-Atmosphäre in einen 300 ml-Parr-Autoklaven eingesetzt wurde. Nach dreimaligem Spülen des Autoklaven mit Stickstoff wurde der CO-Druck auf 40 bar eingestellt. Die Reaktion lief 20 Stunden bei 120°C. Nach Abschluss der Reaktion wurde der Autoklav auf Raumtemperatur abgekühlt und vorsichtig entspannt. Isooctan (100 µl) wurde als interner GC-Standard zugesetzt. Ausbeute und Regioselektivität wurden mittels GC bestimmt.

Die Ergebnisse sind in der nachfolgenden Tabelle dargestellt.

| Ligand | Ausbeute | iso/n |
|---|---|---|
| **2** | 26% | 74/26 |
| **3** (VB) | 16% | 77/23 |
| **7** | 20% | 74/26 |
| **10** (VB) | 0% | N/A |

| | | |
|---|---|---|
| VB: Vergleichsbeispiel | | |

Auch hier zeigen die erfindungsgemäßen Liganden **2** und **7** eine hohe iso/n-Selektivität und eine höhere Ausbeute als die Vergleichsliganden **3** und **10.**

## Patentansprüche

1. Verfahren umfassend die Verfahrensschritte:
a) Vorlegen einer ethylenisch ungesättigten Verbindung;
b) Zugabe eines Monophosphinliganden und einer Verbindung, welche Pd umfasst,
oder Zugabe eines Komplexes umfassend Pd und einen Monophosphinliganden;
c) Zugabe eines aliphatischen Alkohols;
d) Zuführen von CO;
e) Erwärmen des Reaktionsgemisches, wobei die ethylenisch ungesättigte Verbindung zu einem Ester umgesetzt wird;
wobei der Monophosphinligand ausgewählt ist aus Verbindungen gemäß einer der Formeln (1), (2), (7):

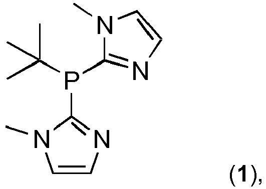

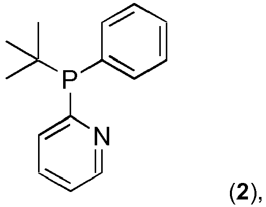

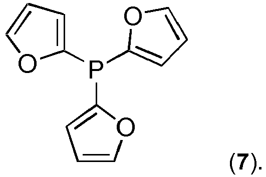

2. Verfahren nach Anspruch 1,
wobei die ethylenisch ungesättigte Verbindung ausgewählt ist aus Ethen, Propen, 1-Buten, *cis-* und/oder *trans*-2-Buten, iso-Buten, 1,3-Butadien, 1-Penten, *cis-* und/oder *trans*-2-Penten, 2-Methyl-1-buten, 3-Methyl-1-buten, 2-Methyl-2-buten, Hexen, Tetramethylethylen, Hepten, 1-Octen, 2-Octen, di-n-Buten, oder Mischungen davon.

3. Verfahren nach einem der Ansprüche 1 oder 2,
wobei die ethylenisch ungesättigte Verbindung 8 bis 12 Kohlenstoffatome umfasst.

4. Verfahren nach einem der Ansprüche 1 bis 3,
wobei die Verbindung, welche Pd umfasst, in Verfahrenschritt b) ausgewählt ist aus Palladiumdichlorid, Palladium(II)-acetylacetonat, Palladium(II)-acetat, Dichloro(1,5-cyclooctadiene)palladium(II), Bis(dibenzylideneaceton)palladium, Bis(acetonitrile)dichloropalladium(II), Palladium(cinnamyl)dichlorid.

5. Verfahren nach einem der Ansprüche 1 bis 4,
wobei der Alkohol in Verfahrensschritt c) ausgewählt ist aus Methanol, Ethanol, 1-Propanol, 1-Butanol, 1-Pentanol, 1-Hexanol, 2-Propanol, *tert*-Butanol, 3-Pentanol, Cyclohexanol, oder Mischungen davon.

6. Verfahren nach einem der Ansprüche 1 bis 5,
wobei der Alkohol in Verfahrensschritt c) ausgewählt ist aus Methanol und Ethanol.

## Claims

1. Process comprising the following process steps:
a) introducing an ethylenically unsaturated compound;
b) adding a monophosphine ligand and a compound which comprises Pd,
or adding a complex comprising Pd and a monophosphine ligand;
c) adding an aliphatic alcohol;
d) supplying CO;
e) heating the reaction mixture, the ethylenically unsaturated compound being reacted to form an ester;
wherein the monophosphine ligand is selected from compounds of one of the formulae (**1**), (**2**), (**7**):

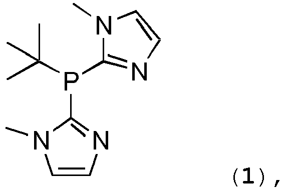

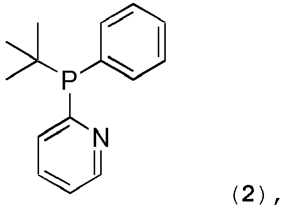

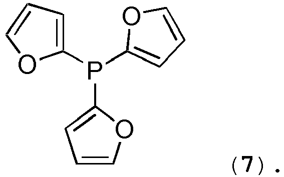

2. Process according to Claim 1,
wherein the ethylenically unsaturated compound is selected from ethene, propene, 1-butene, *cis-* and/or *trans*-2-butene, isobutene, 1,3-butadiene, 1-pentene, *cis-* and/or *trans*-2-pentene, 2-methyl-1-butene, 3-methyl-1-butene, 2-methyl-2-butene, hexene, tetramethylethylene, heptene, 1-octene, 2-octene, di-n-butene, and mixtures thereof.

3. Process according to either of Claims 1 and 2,
wherein the ethylenically unsaturated compound comprises 8 to 12 carbon atoms.

4. Process according to any of Claims 1 to 3,
wherein the compound comprising Pd in process step b) is selected from palladium dichloride, palladium(II) acetylacetonate, palladium(II) acetate, dichloro(1,5-cyclooctadiene)palladium(II), bis(dibenzylideneacetone)palladium, bis(acetonitrile)dichloropalladium(II), palladium(cinnamyl) dichloride.

5. Process according to any of Claims 1 to 4,
wherein the alcohol in process step c) is selected from methanol, ethanol, 1-propanol, 1-butanol, 1-pentanol, 1-hexanol, 2-propanol, *tert*-butanol, 3-pentanol, cyclohexanol, and mixtures thereof.

6. Process according to any of Claims 1 to 5,
wherein the alcohol in process step c) is selected from methanol and ethanol.

## Revendications

1. Procédé comprenant les étapes de procédé suivantes :
a) le chargement d'un composé éthyléniquement insaturé ;
b) l'ajout d'un ligand monophosphine et d'un composé qui comprend du Pd,
ou l'ajout d'un complexe comprenant du Pd et un ligand monophosphine ;
c) l'ajout d'un alcool aliphatique ;
d) l'introduction de CO ;
e) le chauffage du mélange réactionnel, le composé éthyléniquement insaturé étant transformé en un ester ;
le ligand monophosphine étant choisi parmi les composés selon l'une quelconque des formules (**1**), (**2**), (**7**) :

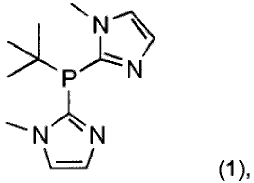

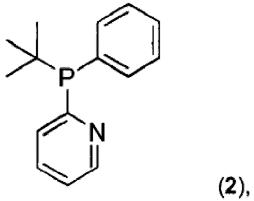

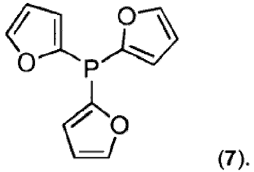

2. Procédé selon la revendication 1, dans lequel le composé éthyléniquement insaturé est choisi parmi l'éthène, le propène, le 1-butène, le *cis-* et/ou le *trans*-2-butène, l'isobutène, le 1,3-butadiène, le 1-pentène, le *cis-* et/ou le *trans*-2-pentène, le 2-méthyl-1-butène, le 3-méthyl-1-butène, le 2-méthyl-2-butène, l'hexène, le tétraméthyléthylène, l'heptène, le 1-octène, le 2-octène, le di-n-butène ou leurs mélanges.

3. Procédé selon l'une quelconque des revendications 1 ou 2, dans lequel le composé éthyléniquement insaturé comprend 8 à 12 atomes de carbone.

4. Procédé selon l'une quelconque des revendications 1 à 3, dans lequel le composé qui comprend du Pd à l'étape de procédé b) est choisi parmi le dichlorure de palladium, l'acétylacétonate de palladium (II), l'acétate de palladium (II), le dichloro(1,5-cyclooctadiène)palladium (II), le bis(dibenzylidène-acétone)palladium, le bis(acétonitrile)dichloropalladium (II), le dichlorure de palladium-(cinnamyle).

5. Procédé selon l'une quelconque des revendications 1 à 4, dans lequel l'alcool à l'étape de procédé c) est choisi parmi le méthanol, l'éthanol, le 1-propanol, le 1-butanol, le 1-pentanol, le 1-hexanol, le 2-propanol, le *tert*-butanol, le 3-pentanol, le cyclohexanol ou leurs mélanges.

6. Procédé selon l'une quelconque des revendications 1 à 5, dans lequel l'alcool à l'étape de procédé c) est choisi parmi le méthanol et l'éthanol.
